# EUROPEAN PATENT SPECIFICATION

(11) **EP 3 336 175 B1**
(45) Date of publication and mention of the grant of the patent: **30.08.2023**
(21) Application number: 16306718.4
(22) Date of filing: 16.12.2016
(51) Int. Cl.: C12N 5/071, C12N 5/0783

(54) **THREE-DIMENSIONAL MODEL OF DEPIGMENTING DISORDER**
DREIDIMENSIONALES MODELL EINER DEPIGMENTIERUNGSSTÖRUNG
MODÈLE TRIDIMENSIONNEL D'UN TROUBLE DE DÉPIGMENTATION

(43) Date of publication of application: 20.06.2018
(73) Proprietor: UNIVERSITE DE BORDEAUX, 33000 Bordeaux (FR); INSERM (Institut National de la Santé et de la Recherche Médicale), 75013 Paris (FR); Centre Hospitalier Universitaire de Bordeaux, 33404 Talence Cedex (FR)
(72) Inventor: BONIFACE, Katia, 33700 MERIGNAC (FR); SENESCHAL, Julien, 33000 BORDEAUX (FR); BERNARD, François-Xavier, 86160 SAINT MAURICE-LA-CLOUERE (FR)
(74) Representative: Cabinet Becker et Associés

(56) References cited:
- WO-A1-2007/061168
- FR-A1- 3 007 037
- US-A- 5 861 153
- US-A1- 2014 348 788
- LI P ET AL: "Interleukin-33 affects cytokine production by keratinocytes in vitiligo.", CLINICAL AND EXPERIMENTAL DERMATOLOGY MAR 2015, vol. 40, no. 2, March 2015 (2015-03), pages 163-170, XP009193970, ISSN: 1365-2230
- RICHMOND JILLIAN M ET AL: "Keratinocyte-Derived Chemokines Orchestrate T-Cell Positioning in the Epidermis during Vitiligo and May Serve as Biomarkers of Disease", JOURNAL OF INVESTIGATIVE DERMATOLOGY, NATURE PUBLISHING GROUP, US, vol. 137, no. 2, 18 November 2016 (2016-11-18), pages 350-358, XP009193975, ISSN: 1523-1747, DOI: DOI:10.1016/J.JID.2016.09.016
- LEI T C ET AL: "A Melanocyte-Keratinocyte Coculture Model to Assess Regulators of Pigmentation in Vitro", ANALYTICAL BIOCHEMISTRY, ELSEVIER, AMSTERDAM, NL, vol. 305, no. 2, 15 June 2002 (2002-06-15) , pages 260-268, XP027227027, ISSN: 0003-2697 [retrieved on 2002-06-15]
- CARLOS R. CAMARA-LEMARROY ET AL: "The Role of Tumor Necrosis Factor-[alpha] in the Pathogenesis of Vitiligo", AMERICAN JOURNAL OF CLINICAL DERMATOLOGY, vol. 14, no. 5, 1 October 2013 (2013-10-01), pages 343-350, XP055359684, US ISSN: 1175-0561, DOI: 10.1007/s40257-013-0039-3

## Description

The present invention relates to the field of tissue engineering, dermatology and cosmetic. The description more specifically relates to a three-dimensional cellular model of depigmenting disorders as well as to products and methods for preparing such a model. It further relates to uses of the model in experimental research, typically for studying pigmentation/depigmentation mechanisms and for the screening of molecules of interest in the prevention or treatment of pathological conditions wherein a depigmenting disorder is observed, in particular vitiligo as well as other inflammatory dermatoses.

### BACKGROUND

Vitiligo is the most common skin depigmenting disorder. The estimated prevalence of vitiligo is around 0.5 % to 1% of the world population. The two sexes are equally affected, and there are no apparent differences in rates of occurrence according to skin type or race. It is clinically characterized by symmetric and bilateral white macules, resulting from a loss of epidermal melanocytes, the pigment-producing cells.

Several mechanisms have been implicated to explain melanocyte disappearance, including genetic predisposition, environmental triggers (such as friction), metabolic alteration, and altered inflammatory and immune responses. Vitiligo is described as an acquired chronic depigmenting disorder of the skin which is associated with autoimmune components involving both CD4⁺ and CD8⁺ T cells. However the phenotype and contribution of effector memory T (TEM) cell subsets remain to date unclear and controversial due to the lack of extensive analysis in models of the disease.

Depigmentation can also occur in several inflammatory skin diseases/dermatoses such as psoriasis, atopic dermatitis, scleroderma, typically cutaneous scleroderma, hypomelanose and leukotrichia. Vitiligo in particular is a stigmatizing disease with major social and psychological impact. Often seen as purely aesthetic, this disorder is indeed responsible for a major impairment of quality of life sometimes comparable to other chronic diseases such as depression. Moreover patients with vitiligo often display other chronic inflammatory disorders such as autoimmune thyroiditis, rheumatoid arthritis and inflammatory bowel diseases. However, despite its prevalence and impact, no specific curative intervention has been reported so far. To date, no treatment has demonstrated efficacy against depigmentation. In addition, there is no treatment allowing the blockade of depigmentation or skin repigmentation, preferably definitive skin repigmentation. Current treatments have overall limited efficacy and include topical steroids or calcineurin inhibitors, systemic steroids and/or UV-light, all of which have potential long-term side-effects.

Despite significant unmet treatment needs, there is currently no cellular model, in particular no *ex vivo* or *in vitro* three-dimensional cellular model, on the market for the specific study of depigmentation mechanisms, in particular depigmentation's pathogenesis, or for the testing of molecules that may be of interest for the prevention, treatment and/or alleviation of symptoms of a depigmenting disorder.

### BRIEF DESCRIPTION OF THE INVENTION

Inventors herein describe for the first time a three-dimensional model of depigmenting disorder allowing the specific study of molecular mechanisms leading to the modulation of pigmentation, typically to depigmentation, and the development of methods for the screening of modulatory molecules in order to identify new drugs of interest and prepare preventive as well as therapeutic compositions, typically compositions capable of preventing or of attenuating depigmentation, in particular of preventing or of attenuating the loss of epidermal melanocytes, or of inducing a repigmentation of the skin.

Inventors indeed discovered the synergistic ability of the particular combination of TNFα and IFNγ to modify a cellular structure comprising a pigmented/melanised epidermis (containing both keratinocytes and melanocytes) in order for it to mimic defects observed in skin of patients affected by a depigmenting disorder, in particular to induce melanocytes detachment from the basal layer of an epidermis. Based on this discovery they have developed a method for producing *in vitro*, *ex vivo* or *in vivo* cellular models, in particular three-dimensional cellular models, mimicking a depigmenting disorder, and herein describe such models.

A first object herein described is thus a cellular model of depigmenting disorder wherein the model i) comprises melanocytes, and ii) has been exposed to interferon gamma (IFNγ) and Tumor necrosis factor alpha (TNFα).

A second object herein described is a three-dimensional cellular model of depigmenting disorder wherein the model i) comprises keratinocytes and melanocytes, and ii) has been exposed to interferon gamma (IFNγ) and Tumor necrosis factor alpha (TNFα).

Another object herein described is a kit comprising IFNγ and TNFα, and its use for preparing *in vitro* or *ex vivo* a three-dimensional cellular model of depigmenting disorder.

Also herein described is the *in vitro*, *ex vivo* or *in vivo* use of a combination of IFNγ and TNFα for preparing a cellular model of depigmenting disorder, in particular a three-dimensional cellular model.

Further herein described is a method of preparing *in vitro*, *ex vivo* or *in vivo* a cellular model of depigmenting disorder, in particular a three-dimensional cellular model, comprising a step of exposing, simultaneously or sequentially, a population of cells comprising at least melanocytes, preferably keratinocytes and melanocytes, or a skin sample, to IFNγ and TNFα. Also described is the use of a cellular model of depigmenting disorder according to the invention for studying *in vitro* or *ex vivo* for example pigmentation/depigmentation mechanisms and potential modulators of these mechanisms. The cellular model can also be used in a method for screening, typically *in vitro* or *ex vivo,* pharmaceutically active molecules suitable for preventing, treating or attenuating a depigmenting disorder, disease or dysfunctional state in a subject in need thereof.

A method for screening, typically *in vitro* or *ex vivo,* pharmaceutically active molecules suitable for preventing, treating or attenuating a depigmenting disorder in a subject in need thereof is also described. This method comprises a step of exposing a cellular model of depigmenting disorder as herein described to a molecule to be tested and a step of evaluating the ability of the tested molecule to inhibit MMP9 expression or function, to inhibit both MMP9 and Janus kinases (JAK) expression or function, in particular the expression or function of a JAK selected from JAK1, JAK2, JAK3, TYK2 and any combination thereof to induce melanin production and/or melanocyte function, to cleave E-cadherin, to increase soluble E-cadherin expression, to increase cytoplasmic redistribution of E-cadherin, to relocalize membrane bound E-cadherin, and/or to stabilize melanocytes to the basement membrane of the epidermis, said ability(ies) indicating that the tested molecule is a pharmaceutically active molecule suitable for preventing, treating or attenuating a depigmenting disorder in a subject in need thereof.

### DETAILED DESCRIPTION OF THE INVENTION

The skin is a large and complex tissue providing a protective interface between an organism and its environment. Epidermis forms its external surface, and is mainly constituted of multiple layers of specialized epithelial cells named keratinocytes. Melanocytes are melaninproducing cells located in the bottom layer of the epidermis and are responsible for the production of melanin: the pigment primarily responsible for skin color. Skin can be injured by many different causes, including microorganisms, chemicals, behaviours, physical injury, ageing, U.V. irradiation, cancer, autoimmune or inflammatory diseases.

The present invention advantageously provides new means for studying and understanding specific mechanisms leading to a disorder of skin pigmentation, typically to a depigmenting disorder, i.e. to a disorder inducing a depigmentation.

A loss of epidermal melanocytes and/or the detection of functional abnormalities of epidermal melanocytes (such as abnormalities of melanocyte adhesion to the basement membrane of epidermis, melanocytes proliferation's abnormalities, increase of inflammatory proteins secretion, abnormalities of melanin and/or melanosomes transfer to keratinocytes) are the pathogenic hallmark of disorders associated with skin depigmentation. Vitiligo, the most common depigmenting disease is associated with complete loss of melanocytes. Factors involved in the initiation of vitiligo remain largely unknown, but likely include genetic predisposition and environmental triggers (such as friction). However, the mechanisms leading to the loss of melanocytes are still in debate. Several theories have been proposed. Melanocyte-intrinsic abnormalities in vitiligo leading to impaired melanocyte degeneration and/or proliferation support the hypothesis that the disease could be due to a primary defect of melanocytes. However, other observations strongly support the role of the autoimmune system, particularly in the chronic and progressive phases of vitiligo. Genome wide association studies have shown that 90% of vitiligo susceptibility loci encode components of the immune system. Furthermore, vitiligo is often associated with autoimmune diseases, and both innate and adaptive immune cells are found in patients' affected skin. Loss of melanocytes could result from cell destruction through melanocyte-specific cytotoxic immune responses (mainly through CD8⁺ cells) and/or melanocyte detachment through a defective adhesion system, also called melanocytorrhagy. E-cadherin, which is the major mediator of human melanocytes adhesion in the epidermis, was recently shown to be absent or discontinuously distributed across melanocyte membranes of vitiligo patients. Such alteration is responsible for melanocytes detachment from the basal layer of the epidermis and their subsequent loss.

TNFα has been largely described for its dual role as both a pro-apoptotic and a pro-inflammatory cytokine. Anti-TNF therapies have proven efficacy in a number of autoimmune and inflammatory disorders, in particular in the field of dermatology, rheumatology, and gastroenterology. Conflicting results have been reported regarding a deregulation of TNFα production in peripheral mononuclear cells and sera from vitiligo patients (Camara-Lemarroy *et al.*). Regarding TNFα secreting cells in patients, cytotoxic CD8⁺ and CD4⁺ T cells have been described as potent producers; yet keratinocytes, fibroblasts, and even melanocytes could be other sources of this cytokine under inflammatory conditions. Regarding the possible involvement of TNFα in vitiligo pathophysiology, *in vitro* studies revealed direct effects of this pro-inflammatory cytokine on melanocyte function. Indeed, TNFα inhibits the expression of microphtalmia associated transcription factor (MITF), essential for melanocyte development, survival, and function, of tyrosinase and tyrosinase related protein 1 (TRP-1), two enzymes involved in melanogenesis. Furthermore, TNFα could enhance T cell trafficking to the skin through upregulation of intercellular adhesion molecule-1 (ICAM-1) expression both in melanocytes and keratinocytes. ICAM-1 is important in the initiation of inflammation and has been detected in melanocytes around active vitiligo patches.

If data from the literature tends to support a role of TNFα in vitiligo, the limited available evaluation of anti-TNF therapies in vitiligo suggests that blocking TNFα is effective at best in halting disease progression but does not induce significant repigmentation.

IFNγ is the most important cytokine related to the Th1 immune response. Binding of this cytokine to its receptor induces the Janus kinase (JAK) / signal transducer and activator of transcription (STAT) pathway, in particular JAK1, JAK2 and STAT1 activation, and regulates the expression of genes/proteins involved in a number of processes, such as defense against pathogens, proliferation, survival/apoptosis, immunomodulation, and leukocyte trafficking. As herein demonstrated, the expression of IFNγ is increased in the skin of patients with vitiligo. Comparison of the transcriptional profile of lesional vitiligo skin with healthy skin revealed an IFNγ specific signature in vitiligo. During disease, IFNγ is mainly produced by cytotoxic CD8⁺ and CD4⁺ T cells.

In the experimental part of the present description, inventors have demonstrated the infiltration of skin inflammatory T cells expressing TNFα and IFNγ in progressive vitiligo and the involvement of TNFα and IFNγ in melanocytes loss through alteration of E-cadherin expression and induction matrix metalloproteinase-9 (MMP9), an enzyme described to cleave E-cadherin (Symowicz et al. Cancer Res 2007). In addition, they have discovered the surprising synergistic action of two cytokines, namely TNFα and IFNγ, in i) downregulating the expression of genes involved in pigmentation signaling, such as in particular MITF, the melanocyte master transcription factor, tyrosinase (TYR), and dopachrome tautomerase (DCT), ii) upregulating the expression of inflammatory genes in melanocytes, including IL-6, TNFα, and intercellular adhesion molecule-1 (ICAM-1), and iii) inducing the production of the CXCR3 ligands CXCL9 and CXCL10. Inventors further demonstrated that TNFα and IFNγ synergistically downregulate the expression of CDH1 transcripts in melanocytes but not in keratinocytes. They showed that TNFα upregulated expression of MMP9 gene in melanocytes and that this effect was also surprisingly potentiated in the presence of IFNγ. In addition, they showed that TNFα upregulated expression of MMP9 gene in keratinocytes.

Inventors herein describe for the first time three-dimensional cellular models of depigmenting disorder. Models herein described comprise at least melanocytes, preferably at least melanocytes and keratinocytes, for example melanocytes, keratinocytes and fibroblasts, and advantageously reproduce features typical of a skin suffering from a depigmenting disorder, for example an inflammatory depigmenting disorder. Preferred features are those whose manipulation is desirable for purposes of research and development of new pharmacological molecules or of new combinations of pharmacological molecules for preventing, treating or alleviating a depigmenting disorder, disease or dysfunctional state, for example an inflammatory depigmenting disorder, disease or dysfunctional state.

A typically preferred feature of the model described by inventors relates to the presence in said model of "detached" melanocytes, i.e. of melanocytes which are detached from the basal layer of the epidermis, and/or from neighboring/adjacent keratinocytes. In a typical model, "detached" melanocytes represent the majority of melanocytes of the cellular model, i.e. more than 50%, for example at least 60%, 70%, 80%, 90%, 95%, 96%, 97%, 98% or 99% of melanocytes.

The number of melanocytes can be easily counted and the proportion of melanocytes detached from the basal layer can be easily determined by the skilled person using conventional microscopy and marking/labeling methods of the art.

The herein described model typically exhibits in addition several (at least two) or all of the herein below described features.

Another typical feature of the model relates to the presence therein of dead (or apoptotic) melanocytes, typically of disrupted melanocytes.

A further feature of the model relates to the analysis of cell death through investigation of the expression of caspase 3 or DNA fragmentation ("TUNEL" assay - Methods Mol Biol. 2002; 203:21-30. TUNEL assay. An overview of techniques. Loo DT).

Another feature of the model herein described relates to a decreased expression of melanin (the pigment produced by melanocyte responsible for the skin coloration) when compared to a portion of the cell structure used to prepare the model which has not been exposed to the combination of TNFα and IFNγ.

An additional feature of the model described by inventors relates to an increase of soluble (cleaved) E-cadherin or of altered membranous E-cadherin expression (concentration) and/or to an increase of membranous E-cadherin cytoplasmic relocation, when compared to a portion of the cell structure used to prepare the model which has not been exposed to the combination of TNFα and IFNγ. Soluble E-cadherin levels can be typically assessed by ELISA in cell free culture supernatants (for example, with a kit from R&D systems). Membrane full length E-cadherin expression can be assessed by immunofluorescence.

A further feature of the herein described model relates to the inhibition of the expression, or to the decreased expression, of Melanocyte Master Transcription Factor (MITF), Tyrosinase (TYR), Cadherin-1 also known as E-cadherin (CDH1), Dopachrome tautomerase (DCT), Tyrosinase Related Protein 1 (TRP1), connective tissue growth factor (CCN2), nephroblastoma overexpressed (CCN3) and Discoidin Domain Receptor family, member 1 (DDR1) genes and/or proteins, preferably of the DCT gene or protein, when compared to a portion of the cell structure used to prepare the model which has not been exposed to the combination of TNFα and IFNγ.

Another feature of the herein described model relates to the stimulation of the expression of interleukin-6 (IL6), tumor necrosis factor (TNF), in particular TNFα, and intercellular adhesion molecule-1 (ICAM-1) genes and/or proteins, when compared to a portion of the cell structure used to prepare the model which has not been exposed to the combination of TNFα and IFNγ.

A further feature of the herein described model relates to the increased expression of C-X-C Motif Chemokine Receptor 3 (CXCR3) ligands such as C-X-C Motif Chemokine ligand 9 (CXCL9) and C-X-C Motif Chemokine ligand 10 (CXCL10).

Assessment of the expression of the herein above identified proteins or genes can be easily performed by the skilled person respectively by immunohistochemistry/immunofluorescence and real time PCR. Chemokines levels can also be assessed by ELISA in cell-free supernatants.

A typical model is a three-dimensional cellular model of depigmenting disorder wherein the model i) comprises keratinocytes and melanocytes and ii) has been exposed to interferon (IFN), in particular interferon gamma (IPNγ), and Tumor necrosis factor alpha (TNFα). The model can further comprise fibroblasts. The model, typically the *in vitro* three-dimensional model containing both melanocytes, keratinocytes, and fibroblasts, or the *ex vivo* model (*ex vivo* culture of a skin biopsy or plasty), can also comprise a cutaneous/skin immune system, i.e. immune cells such as CD8⁺ and CD4⁺ T cells.

A typical cellular model described by inventors is an *in vitro* or *ex vivo* three-dimensional cellular model. Such a cellular model is typically a skin model, an epidermis model, or a tissue model comprising an epidermis and a dermis.

The herein described cellular model is typically prepared from mammal cells, for example from non-human primate (monkeys) cells, from domesticated animals cells (e.g., cows, sheep, pigs, rabbits cats, dogs, and horses cells), or from rodent cells (such as rat or mouse cells). The cellular model is preferably prepared from human cells.

The cellular model can be prepared from cells from a healthy subject or from a patient suffering from a depigmenting disorder or from an inflammatory skin disease, typically an inflammatory skin disease where depigmentation is occurring or associated with depigmentation.

In the context of the invention, the "subject" is an animal, typically a mammal. Examples of mammals include humans and non-human animals such as, without limitation, domesticated animals (e.g., cows, sheep, pigs, rabbits cats, dogs, and horses), non-human primates (such as monkeys), and rodents (e.g., mice and rats). The "subject" is preferably a human being, whatever its gender, age, race or sex.

Typically, the cellular model described by inventors is prepared from cells from a patient suffering from, or diagnosed or identified as suffering from, a depigmenting disorder, disease or dysfunctional state, an inflammatory skin disease, or a depigmenting disorder associated to an inflammatory skin disease (the depigmenting disorder being typically a symptom of the inflammatory skin disease). Preferably, the depigmenting disorder, disease or dysfunctional state is not a depigmenting disorder, disease or dysfunctional state of genetic or infectious origin.

In such a context, the cells used to prepare the model are preferably healthy cells from the patient or in other words cells from a biopsy performed in an unaffected area of the patient, typically a pigmented area of the skin patient.

In a particular example, the cellular model is prepared from cells from a patient suffering from an inflammatory skin disease (also herein identified as "inflammatory dermatose") selected from vitiligo, psoriasis, atopic dermatitis, scleroderma, typically systemic sclerosis, preferably cutaneous scleroderma, hypomelanose and leukotrichia. The patient suffering from the inflammatory skin disease typically has or is at risk of developing a depigmenting disorder.

Psoriasis is a chronic inflammatory multisystem disease, with predominantly skin and joint manifestations, affecting approximately 2% of the population. Almost 90% of affected individuals have psoriasis vulgaris. The major manifestation of psoriasis is chronic inflammation of the skin. It is characterised by disfiguring, scaling and erythematous plaques that may be painful or often severely pruritic. Psoriasis is a chronic disease that waxes and wanes during a patient's life time. Plaque psoriasis is the most common form, affecting approximately 80% to 90% of patients. Plaque psoriasis manifests as well-defined, sharply demarcated, erythematous plaques varying in size from 1 cm to several centimeters. Patients may have involvement ranging from only few plaques to numerous lesions covering almost the entire body surface. The inflammatory response developed in psoriasis can lead to skin depigmentation according to the same ranging. This manifestation alters the patients' life quality in the same way vitiligo does, the unaesthetic manifestations leading to depressive disorder or social withdrawal. Moreover spontaneously occurring vitiligo can be associated with psoriasis.

Atopic dermatitis (AD) is a chronic relapsing inflammatory skin disorder that affects approximately 2% to 5% of the adult population in the western world. The disease involves complex combinatorial pathogenic effects, multiple susceptibility genes, environmental triggers and disruption of the epidermal barrier. Clinically, AD can result in impairment of skin function, with intense and bothering itching leading to considerable loss of sleep and poor quality of life that is usually associated with disease severity. The inflammatory response developed in Atopic Dermatitis can lead to skin depigmentation according to the same ranging. This manifestation alters the patients' life quality in the same way vitiligo does, the unaesthetic manifestations leading to depressive disorder or social withdrawal. Moreover spontaneously occurring vitiligo can be associated with atopic dermatitis.

Scleroderma is an inflammatory disease. There are two forms of the disease: i) localized scleroderma (called "morphea"), involves isolated patches of hardened skin with no internal involvement and ii) systemic scleroderma (also called "systemic sclerosis") comprising two subgroups based on the extent of skin involvement (herein generally identified as "cutaneous scleroderma"): the subgroups are diffuse cutaneous scleroderma and limited cutaneous scleroderma. Systemic sclerosis can be associated with progressive visceral organs defects, including the kidneys, heart, lungs and gastrointestinal tract. Skin manifestations are progressive skin tightness and induration and prominent skin pigmentary changes (both hyperpigmentation and hypopigmentation). Those skin manifestations, as discussed with psoriasis and vitiligo, are responsible for a major alteration of patients' quality of life.

In a preferred embodiment, the cellular model herein described is prepared from healthy cells from a healthy patient.

The herein described cellular model is typically prepared from a population of cells from a subject as herein defined who is either a healthy subject or a patient suffering from a disease or disorder as herein described. The population of cells comprises at least melanocytes, preferably at least melanocytes and keratinocytes, and can also contain fibroblasts and/or immune cells from the cutaneous/skin immune system such as CD8⁺ and CD4⁺ T cells. The population of cells can be prepared from cells extracted from a biological sample (biopsy or plasty) of the subject. The biological sample is typically a (healthy) skin sample obtained by surgery. The healthy skin sample is preferably a foreskin sample, but it can also be an abdominal or mammary/breast skin sample. The skin sample is typically a healthy skin sample obtained by surgery. A skin biopsy can also be obtained from a patient suffering from an inflammatory depigmenting disorder. The skin sample may vary from 1 to 50 cm² in size. The skin biopsy is preferably from 3 to 6 mm in diameter.

The population of cells can be used to prepare a reconstructed cellular structure such as a skin, an epidermis or a tissue-like or skin-like structure containing an epidermis and a dermis. Such a population of cells or reconstructed cellular/cell structure is typically exposed to a culture medium.

Culture medium usable in the context of the present invention are standard culture medium classically used by the skilled person such as Epilife^{®} Medium, CnT-Prime 3D Barrier^{®} Medium (CELLnTEC) and 3D-Skin Medium^{®} (PrimCells).

A culture medium usable in the context of the invention is for example a complete EpiLife^{®} Medium, such as for example the cascade biologies^{®} base medium reference M-EPI-500-CA.

The culture medium is typically supplemented for example with insulin, vitamin C, keratinocyte growth factor (KGF) and/or CaCl₂.

Preferably, the culture medium is supplemented with 5 µg/ml of bovine insulin, 50 µg/ml of vitamin C, 3 ng/ml of keratinocyte growth factor (KGF), and 1,5 mM of CaCl₂. Even more preferably, the culture medium comprises, or consists in, 20% pituaray extract, 5µg/ml bovin transferrin bovin, 0.2ng/ml recombinant human EGF (supplements Epilife), 5 µg/ml of bovine insulin, 50 µg/ml of vitamin C, 3 ng/ml of keratinocyte growth factor (KGF), and 1,5 mM of CaCl₂.

In a particular aspect, when the model is a skin model, typically an *ex vivo* skin model, the culture medium comprises DMEM, fetal calf serum, typically 10% of fetal calf serum, and hydrocortisone, typically 0,4 µg/ml of hydrocortisone (such as a Bioalternatives^{®} culture medium).

Whatever their origins, inventors herein reveal for the first time that cells or cell structures used to prepare the model of the invention are to be exposed specifically to both interferon gamma (IFNγ) and Tumor necrosis factor alpha (TNFα).

The use of a combination of IFNγ and TNFα for preparing a cellular model of depigmenting disorder, in particular a three-dimensional cellular model, is thus herein described, in particular the *in vitro* or *ex vivo* use of such a combination. IFNγ and TNFα can for example be introduced into the culture medium.

As well, a composition and a kit comprising IFNγ and TNFα are herein disclosed.

The composition comprising IFNγ and TNFα can further comprise a pharmaceutically acceptable carrier or support, typically a culture medium as herein above described.

Culture media usable in the context of the present invention for a skin sample are standard culture media classically used by the skilled person such as Dulbecco's minimal essential medium (DMEM) supplemented or not with serum.

A particular kit comprising IFNγ and TNFα further comprises a culture medium as herein described and optional supplements thereof such as for example epidermal growth factor (EGF), transforming growth factor-α (TGFα), keratinocyte growth factor (KGF), the medium and each of the supplements being present in distinct containers.

Another particular kit comprising IFNγ and TNFα further comprises a culture medium as herein described and optional supplements thereof such as for example Epilife^{®} Medium supplements and/or insulin, the medium and each of the supplements being preferably present in distinct containers.

Also disclosed is the use of a kit as herein described comprising IFNγ and TNFα for preparing *in vitro* or *ex vivo* a cellular model of depigmenting disorder, in particular a three-dimensional cellular model.

Inventors further herein provide a method of preparing, typically *in vitro* or *ex vivo,* a cellular model of depigmenting disorder, in particular a three-dimensional cellular model of depigmenting disorder. This method comprises a step of exposing a population of cells or cell structure, typically a reconstructed pigmented/melanised epidermis (containing or not dermis), comprising at least melanocytes, preferably at least keratinocytes and melanocytes, but could also contain melanocytes, keratinocytes and fibroblasts, or a (whole) skin sample, to IFNγ and TNFα.

Cells or cell structures, typically cultured cells or cultured cell structures, used to prepare a model of the invention can be exposed to IFNγ and TNFα simultaneously or sequentially and in any order, i.e. IFNγ before TNFα or TNFα before IFNγ. They are typically exposed to IFNγ and TNFα at a concentration and for a time sufficient, preferably about 24 hours, to allow the cell structure or biopsy to acquire features (such as anyone of those described previously) of a skin suffering from a depigmenting disorder, typically from vitiligo, preferably at a concentration and for a time sufficient for the cell structure to mimic a depigmenting disorder, typically vitiligo. A preferred acquired feature is "melanocytes detachment".

The method of the invention typically comprises a step of exposing cells to a culture or culture medium, wherein the medium comprises IFNγ and TNFα. In a particular embodiment, IFNγ and TNFα are respectively present in a concentration ranging from about 0.1 to 50 ng/ml of medium. Preferably, IFNγ and TNFα are each present at a concentration of about 10 ng/ml of medium.

Typically, cells are exposed to a concentration of IFNγ ranging from about 0.1 to 50 ng/ml of medium, typically from about 1 to 50 ng/ml of medium, from about 5 to 30 ng/ml of medium, from about 5 to 20 ng/ml of medium, from about 5 to 15 ng/ml or from about 8 to 12 ng/ml of medium, for example to a concentration of IFNγ of about 1, 2, 3, 4, 5, 6, 7, 8, 9, 10, 11, 12, 13, 14, 15, 16, 17, 18, 19, 20, 21, 22, 23, 24 or 25 ng/ml of medium, and to a concentration of TNFα ranging from about 0.1 to 50 ng/ml of medium, typically from about 5 to 30 ng/ml of medium, from about 5 to 20 ng/ml of medium, from about 5 to 15 ng/ml or from about 8 to 12 ng/ml of medium, for example to a concentration of TNFα of about 1, 2, 3, 4, 5, 6, 7, 8, 9, 10, 11, 12, 13, 14, 15, 16, 17, 18, 19, 20, 21, 22, 23, 24 or 25 ng/ml of medium. Cytokines are diluted in the medium which is typically a culture medium or a culture medium as herein described.

In another particular embodiment, the medium comprises IFNγ, TNFα and insulin.

Cells are typically exposed to each of IFNγ and TNFα for a period varying between about one hour and 6, 12, 24, 48, 72, 96 or 120 hours, preferably 24, 48 or 72 hours. Preferably, cells are exposed to each of IFNγ and TNFα for a period of 24h.

The model of depigmenting disorder of the invention can be used advantageously for studying, typically for studying *in vitro* or *ex vivo,* pigmentation/depigmentation mechanisms as well as (potential) modulators of these mechanisms.

As used herein, the term "modulator" is defined as any molecule able to modulate (activate or inhibit) skin pigmentation or depigmentation. The modulator is typically a pharmaceutically active molecule.

In a preferred embodiment, the model of depigmenting disorder of the invention is used in a method for screening and studying *in vitro* or *ex vivo* pharmaceutically active molecules suitable for preventing, treating or attenuating a depigmenting disorder in a subject in need thereof.

A method for screening, typically for screening *in vitro* or *ex vivo,* pharmaceutically active molecules suitable for preventing, treating or attenuating a depigmenting disorder in a subject in need thereof is further herein described. This method comprises a step of exposing a cellular model of depigmenting disorder as herein described to a molecule to be tested and a step of evaluating the ability of the tested molecule to inhibit MMP9 expression or function, to inhibit both MMP9 and Janus kinases (JAK) expression or function, in particular the expression or function of a JAK selected from JAK1, JAK2, JAK3, TYK2 and any combination thereof to induce melanin production and/or melanocyte function, to cleave E-cadherin, to increase soluble E-cadherin expression, to increase cytoplasmic redistribution of E-cadherin, to relocalize membrane bound E-cadherin, and/or to stabilize melanocytes to the basement membrane of the epidermis, said ability(ies) indicating that the tested molecule is a pharmaceutically active molecule suitable for preventing, treating or attenuating a depigmenting disorder in a subject in need thereof.

The candidate (test) pharmacological molecules/agents can be derived for example from combinatorial peptide libraries, combinatorial chemical compound libraries, and natural products libraries. Molecules which can be tested on the cellular model of the invention are preferably selected for example from monoclonal antibodies, aptamers, spiegelmers, inhibitory nucleic acid sequences, small molecules. In a particular example, these molecules are directed against MMP9. Convenient reagents for such assays are known in the art.

The molecule to be tested can be introduced, typically diluted, in the culture medium into which the model is maintained, for example immersed, or directly applied on the upper cell layer of the model, typically on the epidermis or dermis.

The molecule effects are typically studied in order to evaluate its potential therapeutic, typically dermatological, or cosmetic effect.

The molecule to be tested can be introduced before, at the same time, or after the introduction of TNFα and IFNγ.

To evaluate the potential therapeutic effect of a test molecule, the localization of melanocyte within the epidermis can be assessed, typically the detached (from the basal layer of the epidermis) melanocytes and attached melanocytes present in the epidermis, can be respectively quantified, and compared to the condition where only TNFα and IFNγ are present (positive control of depigmentation). Evaluation/quantification of soluble E-cadherin levels and/or membrane bound E-cadherin expression and/or quantification of melanin content can also be assessed and compared to a positive control. E-cadherin expression in a skin sample is classically assessed by immunofluorescence, immunohistochemistry, or western blot. Soluble E-cadherin levels are classically determined by ELISA

Typically, pluralities of assay mixtures are run in parallel with different agent concentrations to obtain a different response to the various concentrations. Typically, one of these concentrations serves as a negative control, i.e., at zero concentration of agent or at a concentration of agent below the limits of assay detection.

The examples, which follow, and their corresponding figures illustrate the invention without limiting the scope thereof.

### FIGURES

**Figure 1****. Prominent infiltration of skin inflammatory T cells expressing TNFα and IFNγ in progressive vitiligo.** T cells were isolated from perilesional skin of patients with a progressive (n=10) or a stable vitiligo (n=6). Results were compared to healthy controls (n=6). Analysis of IFNγ, TNFα, or IFNγ/TNFα producing skin infiltrating CD4 and CD8 T cells by flow cytometry. Each symbol represents one specimen. The mean + s.e.m. is shown .^{∗}p<0.05, ^{∗∗}p<0.01, using a Mann Whitney test
**Figure 2****. TNFa and IFNγ: two major cytokines involved in melanocyte inhibition**
   **A-B.** Primary cultures of normal human epidermal melanocytes were stimulated in the presence or absence of the indicated cytokines for 24h (IL-17, IL-1β, TNFα, IFNγ: 20ng/ml; TGFβ: 5ng/ml). Real-time analysis of the indicated gene expression. Results are expressed as a fold decrease below control culture (no stimulation). GAPDH was used as a housekeeping gene. Data are representative of three independent experiments.

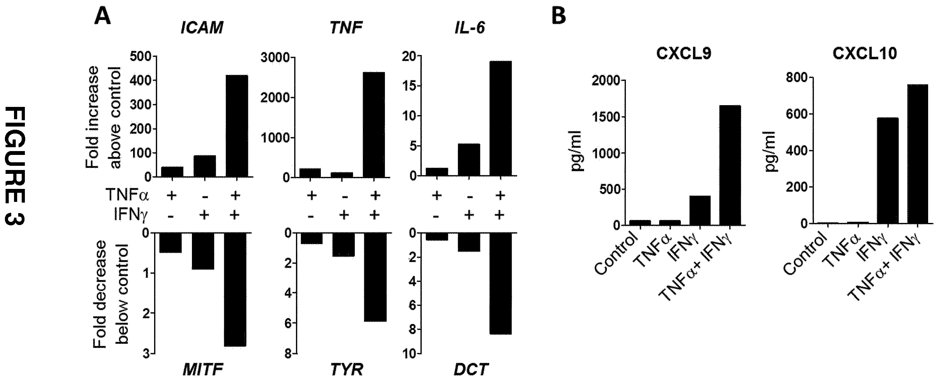

**Figure 3****. Involvement of TNFα and IFNy in the inflammatory response and depigmentation.**
   A-B. Primary cultures of normal human epidermal melanocytes were stimulated in the presence or absence of 20ng/ml TNFα and/or IFNγ for 24h (A) or 48h (B). A. Real-time analysis of the indicated gene expression. Results are expressed as a fold increase above control culture (no stimulation) for upregulated genes (upper panel) or fold decrease below control culture for downregulated genes (lower panel). GAPDH was used as a housekeeping gene. **B.** Measurement of CXCL9 and CXCL10 secretion in cell-free supernatants by ELISA.

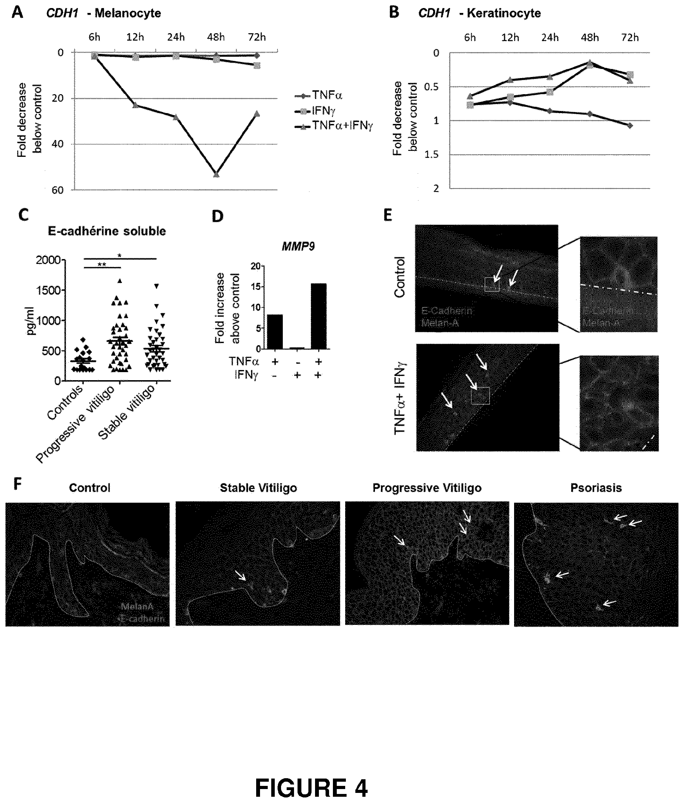

**Figure 4****. Involvement of TNFα and IFNγ in the inflammatory response and melanocyte loss.**
   **A-B.** Primary cultures of normal human epidermal melanocytes (A) or keratinocytes (B) were stimulated in the presence or absence of 20ng/ml TNFα and/or IFNγ for the indicated time. Real-time analysis of *CDH1* gene expression. GAPDH was used as a housekeeping gene. **C.** Assessment of soluble E-cadherin levels in sera from healthy controls (n=20) or patients with a progressive (n=40) or a stable (n=40) vitiligo. Data are representative of three (A-D) or two (E) independent experiments. **D.** Real-time analysis of *MMP9* gene expression in melanocytes treated 24h with or without 20ng/ml of TNFα and/or IFNγ. GAPDH was used as a housekeeping gene. **E.** Reconstructed human epidermis containing melanocytes were stimulated in the presence or absence of 20ng/ml of TNFα and IFNγ for 24h. Immunofluorescence microscopy analysis of melan-A (red) and E-cadherin (green) expression, Magnification 400x. **E.** Immunofluorescence microscopy analysis of melan-A (red) and E-cadherin (green) expression in healthy skin, perilesional skin of patients with stable or progressive vitiligo, and lesional psosiatic skin. Magnification 400x. White arrows show melanocytes that are detached from the basal layer of the epidermis.

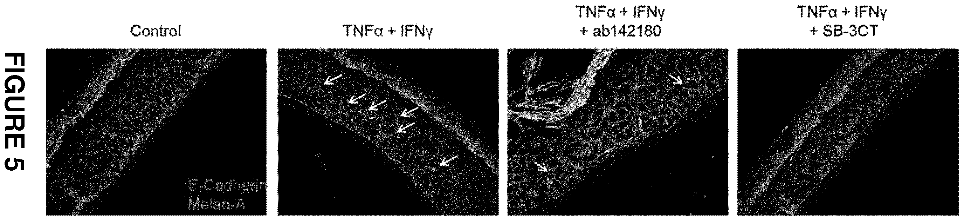

**Figure 5****. TNFα and IFNγ -induced melanocyte loss is mediated through MMP9.**
   Reconstructed human epidermis containing melanocytes were stimulated 24h with or without 20ng/ml of TNFα and IFNγ in the presence or absence of 10µM of MMP9 inhibitor ab142180 or SB-CT. Immunofluorescence microscopy analysis of melan-A (red) and E-cadherin (green) expression, Magnification 400x. White arrows show melanocytes that are detached from the basal layer of the epidermis.

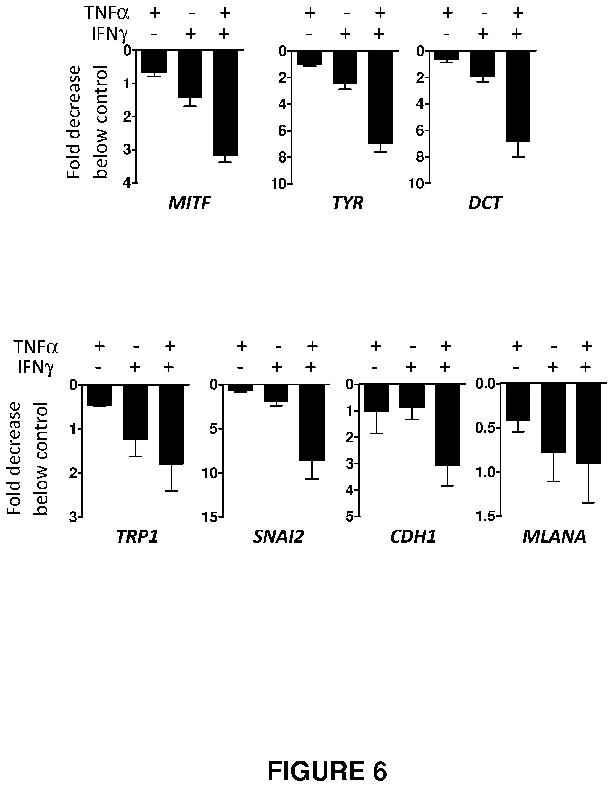

**Figure 6****. Involvement of TNFα and IFNγ in melanocyte loss.**
   Primary cultures of melanocytes were stimulated in the presence or absence of 20ng/ml of TNFα and IFNγ. Analysis using quantitative RT-PCR of the expression of the indicated genes after 24h of stimulation. The gene encoding GAPDH is used as a control gene. Results show the expression ratio between stimulated cells and control cells in the absence of cytokines [mean + standard error of the mean (SEM) of 3 experiments performed on 3 distinct samples]. Inventors observed a synergistic effect of the TNFα and IFNγ cocktail in the inhibition of genes encoding proteins involved in melanocyte function, melanogenesis and melanocytes adhesion.

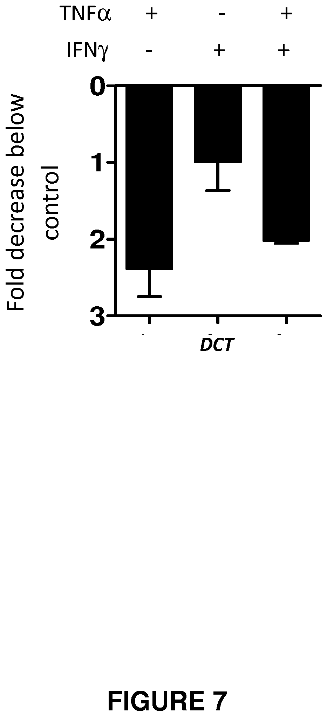

**Figure 7****. Involvement of TNFα and IFNγ in melanocyte loss.**
   Reconstructed human epidermis containing melanocytes were stimulated in the presence or absence of 20ng/ml of TNFα and IFNγ. Analysis using quantitative RT-PCR of the DCT gene expression after 24h of stimulation. The genes encoding GAPDH and β-glucuronidase are used as control genes. Results show the expression ratio between the stimulated epidermis and the control epidermis without cytokines [mean + standard error of the mean (SME) of 2 experiments performed on 2 different samples]. Inventors observe a DCT gene inhibition in presence of TNFα. This effect is not modified by addition of IFNγ.

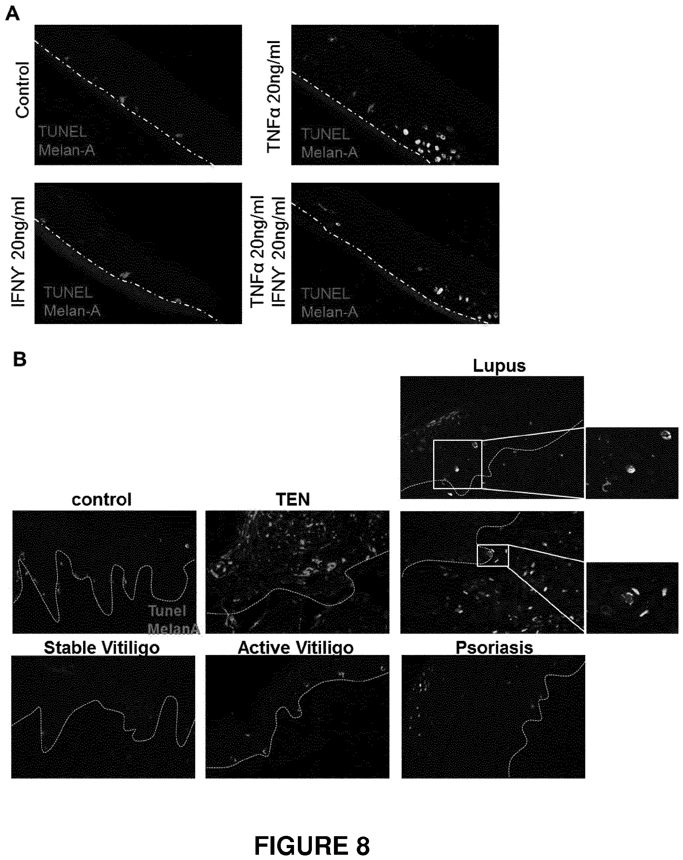

**Figure 8****. Cell death is not a primary mechanism involved in melanocyte loss.**
   (A-B). Reconstructed human epidermis containing melanocytes were stimulated in the presence or absence of 20ng/ml of TNFα and IFNγ for 24h. Analysis of Melan-A expression (red color reveals melanocytes) using immunofluorescence and detection of apoptotic cells (green) using TUNEL technique (results representative of 2 experiments performed on 2 distinct samples). Results show that TNFα and/or IFNγ have no effect on melanocytes apoptosis. A slight pro-apoptotic effect of TNFα on keratinocytes is observed. (B) Analysis of Melan-A expression (red color reveals melanocytes) using immunofluorescence and detection of apoptotic cells (green) using TUNEL technique in healthy skin (control), perilesional skin of patients with stable or progressive vitiligo, lesional skin of patients with psoriasis or lupus. Magnification 400x. TEN is a positive control for apoptosis. Results show that in vitiligo patients (with a stable or active disease), detached melanocytes do not enter apoptosis, emphasizing that melanocyte death is not the primary mechanism involved in their loss.

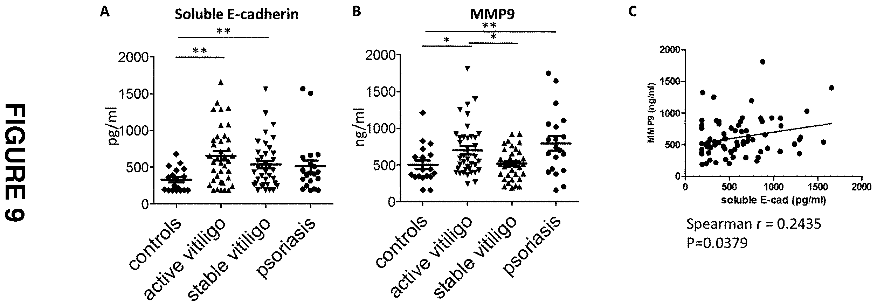

**Figure 9****. MMP9 and soluble E-cadherin levels in vitiligo patients' sera.**
   (A-B) Levels of soluble E-cadherin (A) and MMP9 (B) were determined by ELISA in the serum of patients with vitiligo (stable and progressive) or psoriasis, and in the serum of healthy controls. (C) Serum levels of MMP9 were correlated to serum levels of soluble E-cad in vitiligo patients (Spearman test p<0,05). Inventors show a significant increase of soluble E-cadherin and MMP9 levels in active and stable vitiligo patient's serum compared to healthy controls. Levels of MMP9 were significantly higher in vitiligo patients with active disease. In addition, a positive correlation was observed between MMP9 and soluble E-cadherin levels in vitiligo patients.

### EXAMPLES

### 1/ Increased secretion of TNFα and IFNγ by skin infiltrating T cells in vitiligo

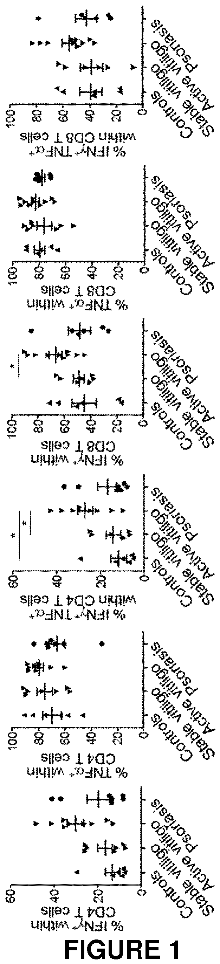

Inventors extracted T cells from perilesional skin of vitiligo patients with a stable or a progressive disease in order to analyze their propensity to produce inflammatory cytokines, in particular TNFα and IFNγ, two cytokines previously identified as potentially involved in disease development (reviewed in Boniface et al. G. Ital. Dermatol. Venereol 2016). Results were compared to those obtained from healthy individuals (Figure 1). They show that CD4 and CD8 T cells extracted from the skin of vitiligo patients with active disease produce higher levels of the type-1 related cytokine IFNγ and TNFα, also known to be secreted by most of T cell subsets, suggesting their involvement in melanocyte loss.

### 2/ TNFα and IFNγ: two major cytokines involved in melanocyte inhibition

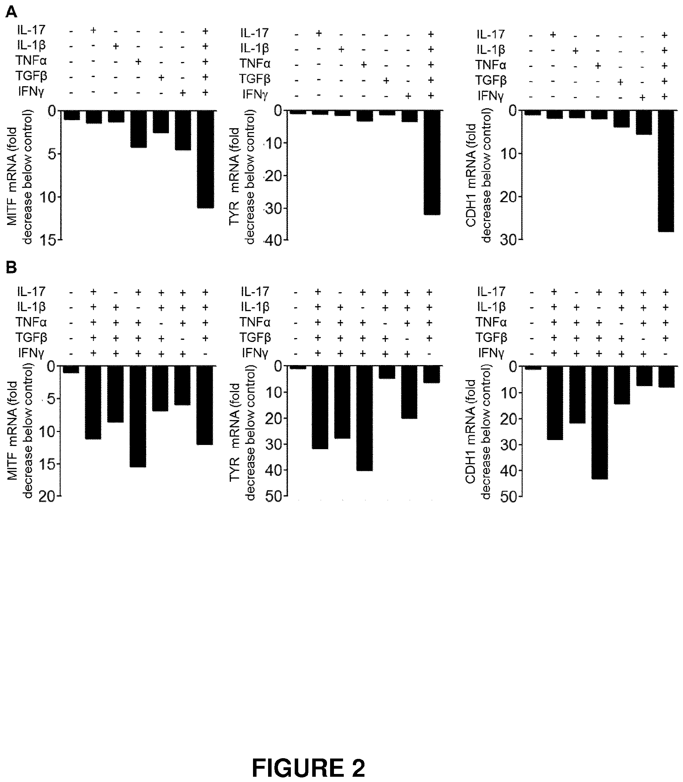

Inventors next assessed the biological activities of pro- or anti-inflammatory cytokines on the expression of genes involved in melanocyte function, such as microphthalmia associated transcription factor (MITF, the melanocyte master transcription factor) and tyrosinase (TYR, involved in melanogenesis), as well as E-cadherin encoding gene (CDH1), a protein critical for melanocyte adhesion to keratinocytes and to the epidermis basal membrane. More precisely, they evaluated the effects of TNFα, IFNγ, IL-17, IL-1β, and TGFβ, alone or in combination, on primary cultures of human epidermal melanocytes (Figure 2). IFNγ or TNFα inhibited the expression of genes involved in melanocyte differentiation, function, melanin synthesis, and melanocyte adhesion (Figure 2A); TGFβ was also able to downregulate melanocyte function, although at a weaker level, whereas IL-17 and IL-1β had no or little effect. Strikingly, the combination of the 5 cytokines resulted in a robust inhibition of the studied genes. Inventors more specifically identified TNFα and IFNγ as the most potent cytokines that inhibit melanocyte function and adhesiveness (Figure 2B).

### 3/ TNFα and IFNγ act synergistically on melanocytes to inhibit pigmentation and induce inflammatory mediators

Inventors evaluated the capability of TNFα and IFNγ to act in concert to modify melanocyte function. As shown in Figure 3 and Figure 6, TNFα and IFNγ synergized to downregulate the expression of genes involved in pigmentation signaling, such as MITF, the melanocyte master transcription factor, tyrosinase (TYR), and dopachrome tautomerase (DCT). TNFα and IFNγ also synergized to upregulate the expression of inflammatory genes in melanocytes, including IL-6, TNFα, and intercellular adhesion molecule-1 (ICAM-1), and they also induced production of the CXCR3 ligands CXCL9 and CXCL10 (Figure 3B). These results reveal for the first time that a melanocyte can become an inflammatory cell under the influence of type-1-related cytokines and could amplify the inflammatory response observed in vitiligo.

### 4/ TNFα and IFNγ induce melanocyte detachment through E-cadherin alteration and induction of MMP9

Inventors showed that TNFα and IFNγ have a synergistic effect to downregulate the expression of CDH1 transcripts in melanocytes (Figure 4A), but not in keratinocytes (Figure 4B). Such effects are of interest as E-cadherin, which is the major mediator of human melanocytes adhesion, was recently shown to be discontinuously distributed across melanocyte membranes of vitiligo patients (Wagner et al. J Invest Dermatol. 2015). E-cadherin is a transmembrane protein existing in two forms: the full length E-cadherin and soluble E-cadherin (David et al. Cancer Res 2012). The soluble form has been shown to be upregulated in the sera of patients with autoimmune diseases (Jin et al. J Rheumatol 2013; Matsuoyoshi et al. Br J Dermatol 1995). Inventors determined the levels of soluble E-cadherin in sera of vitiligo patients. As shown in Figure 4C, levels of soluble E-cadherin were significantly higher in vitiligo patients compared to healthy controls. In addition, such levels tend to correlate with disease activity indicate that levels of soluble E-cadherin can be used as a marker for disease activity. Several enzymes can cleave full length E-cadh and lead to the release of its soluble form, including matrix metalloproteinase MMP9 (aka gelatinase) (Symowicz et al. Cancer Res 2007). Inventors showed that MMP9 levels were significantly higher in serum of patients with vitiligo than in healthy controls (Figure 8). MMP9 levels were significantly higher in patients with active disease compared to stable vitiligo. Strikingly, they observed a positive correlation between MMP9 and soluble E-cadherin levels in vitiligo patients. Importantly, inventors showed that TNFα upregulated expression of MMP9 gene in melanocytes (Figure 4D); such effect was potentiated in the presence of IFNγ. These results indicate that MMP9 is involved in the up-regulation of soluble E-cadherin in vitiligo.

Next, to better characterize the biological activities of TNFα and IFNγ on melanocyte adhesion, inventors prepared an *in vitro* 3D cellular model of containing both melanocytes and keratinocytes. Strikingly, they demonstrated that the combined activities of TNFα and IFNγ induce melanocyte detachment from the model basal layer, at least in part mediated through alteration of E-cadherin expression (Figure 4E). A decrease in DCT, a melanogenesis-associated gene, was also observed (Figure 7). Such phenotype clearly resembles the one observed in perilesional skin of vitiligo patients (with a stable or a progressive disease), where melanocytes are detached from the basal layer of the epidermis (Figure 4F). Interestingly, such phenomenon is also observed in psoriasis (Figure 4F), emphasizing that melanocyte adhesion defect can be extended to other skin inflammatory conditions like psoriasis. Moreover, the inventors showed that TNFα and IFNγ, alone or in combination, have little or no effect on melanocyte apoptosis (Figure 8A). These data are in line with the observation that melanocytes do not undergo apoptosis in vitiligo patients perilesional skin (Figure 8B), emphasizing that melanocytes' detachment is the primary mechanism involved in melanocyte loss in vitiligo.

Altogether, these results are of major interest and identify a new mechanism leading to melanocyte loss, with a critical role of TNFα and IFNγ. In addition, this *in vitro* model of depigmentation can advantageously be used to test new therapeutic targets in vitiligo patients, as well as in patients with depigmenting disorders associated with inflammatory skin diseases.

### 5/ MMP9 inhibition prevents TNFα and IFNγ-induced depigmentation through melanocyte stabilisation to the basal membrane of the epidermis

As discussed above, TNFα and IFNγ induce MMP9 gene expression in melanocyte. Inventors used two commercially available synthetic MMP9 inhibitors, SB3CT (a non-selective MMP2 and MMP3 inhibitor) and a selective MMP9 inhibitor (ab142180), to assess whether such inhibition could inhibit TNFα and IFNγ effects on depigmentation. *In vitro* reconstructed human epidermis containing melanocytes were stimulated with these two cytokines in the presence or absence of the two different MMP9 inhibitors for 24h (Figure 5). The combination of TNFα and IFNγ induced melanocyte detachment and E-cadherin inhibition. Importantly, such effect was strongly inhibited in the presence of either MMP9 inhibitor, as MMP9 inhibition led to melanocyte stabilisation to the basal membrane of the epidermis (Figure 5). These results are the first to demonstrate that IFNγ and TNFα effect on melanocyte adhesion is mediated through MMP9.

All together, these results support the concept that inhibiting MMP9 represent an attractive and novel strategy to prevent melanocyte loss in vitiligo, but also in depigmentation occurring in skin inflammatory disorders such as psoriasis, atopic dermatitis, scleroderma, hypomelanose or leukotrichia.

### 6/ Example of a method of preparing a reconstructed depigmented melanized epidermis model

Pigmented epidermis (RHEm) are reconstructed from normal human keratinocytes (NHEK) and from normal human melanocytes (NHEM), previously isolated from preputial samples.

### 1. RHEm treatments

After 10 days of reconstruction (D10), reconstructed human pigmented epidermis (RHEm) are placed in 6 wells plate (NUNC reference 140675) in 2 ml of culture medium without hydrocortisone to which 10 ng/ml of interferon (IPN)-γ (R&D systems) and 10 ng/ml of "tumor necrosis factor (TNF)-α" (R&D systems) are added. A control condition without cytokines addition is performed. Each experimental condition is performed in duplicate or triplicate.

Composition of the culture medium without hydrocortisone: complete EpiLife^{®} Medium (cascade biologics ^{®} base medium reference M-EPI-500-CA + supplements reference S-001-K among which IGF-1 and hydrocortisone aliquots have not been used) supplemented with:
- 5 µg/ml of bovine insulin (Sigma Aldrich 11882)
- 50 µg/ml of vitamin C (Sigma Aldrich A4403)
- 3 ng/ml of keratinocyte growth factor (KGF) (Millipore GF008)
- 1,5 mM of CaCl₂ (Sigma Aldrich C7902).

After a 24h incubation period, the inserts containing the reconstructed epidermis are collected with pliers, left on a towel in order to remove residual liquid under the insert, then the reconstructed epidermis were cut from the support with a scalpel, put in a formaldehyde solution before dehydration, and then embedded in paraffin.

### 2. Analysis of melanocytes detachment

5 µm sections of paraffin inclusions are deposited on slides for labelling by immunofluorescence to reveal MelanA and E-Cadherin according to the following protocol:
**1- Deparaffinization**

| | |
|---|---|
| Placing the slides on a hot plate at 70°C | 10 min |
| Successive xylene baths | 3 x 5 min |

**2- Hydration**

| | |
|---|---|
| Ethanol 100° | 1 min |
| Ethanol 100° | 1 min |
| Ethanol 95° | 1 min |
| Ethanol 70° | 1 min |
| Ethanol 50° | 1 min |
| Distilled water | 1 min |
| PBS or TBS | 5 min |

**3- Antigen accessibility**
Use of the Decloacking Chamber device BIOCARE Medical
Place the slides into the device at pH8
Launch the program (98°C - 20 min)
Wash with TBS (Tris buffer saline) 0.1% Tween at a temperature equal to cooling temperature (60-70°C).

Allow to come back to room temperature

| | | |
|---|---|---|
| **4-** | Wash with TBS 0.1% tween | 3 x 5 min |
| **5-** | Delimit the deposition areas with a hydrophobic pen | |
| **6-** | Put the primary anti-MELANAA antibody **(1)** at 1/100 dilution and the anti-E-CADHERIN antibody **(2)** at 1/100 dilution in the buffer **(3)** Incubate overnight at 4°C | |
| **7-** | The day after: wash with TBS 0.1% tween Put the secondary antibody A555 **(4)** at 1/100 dilution in the buffer **(3)** + A 488 **(5)** at 1/100 dilution in the buffer **(3)** | 3 x 5 min 60 min |
| **8-** | Wash with TBS 0.1% tween | 3 x 5 min |

Assemble slide with medium assembly with "DAPI" **(6)** away from light.
**(1):** DAKO Melan-A Clone A103 Isotype IgG1, Kappa (ref: M 7196)
**(2):** Abeam rabbit anti-E-cadherin antibody (ref: ab 15148)
**(3):** DAKO Antibody Diluent with background reducing components (ref: S3022)
**(4):** Invitrogen Alexa 555 goat anti mouse IgG (ref: A21422)
**(5):** Invitrogen Alexa 488 rabbit IgG(H+L) (ref: A21441) or Novus (ref: NBP 1-72944)
**(6):** Molecular Probes Prolong gold antifade reagent with DAPI (ref: P36935)
Then reading with a fluorescence microscope for the analysis of melanocytes detachment.

Conclusion: Inventors developed *in vitro* and *ex vivo* human 3D models that mimic depigmentation observed in inflammatory depigmenting disorders, in particular vitiligo, but also in skin inflammatory disorders where depigmentation can occur, such as psoriasis, atopic dermatitis, scleroderma, hypomelanose and leukotrichia. These in vitro and/or *ex vivo* models can be used to model inflammatory depigmentation to study the involved mechanisms and/or to test the potential of pharmaceutically active molecules to prevent, treat or attenuate a depigmenting disorder.

### REFERENCES

- Boniface K, Taieb A, Seneschal J. New insights into immune mechanisms of vitiligo. G. Ital. Dermatol. Venereol. 2016;151(1):44-54.
- Camara-Lemarroy CR, Salas-Alanis JC. The role of tumor necrosis factor-α in the pathogenesis of vitiligo. Am J Clin Dermatol. 2013;14:343-50.
- David JM, Rajasekaran AK. Dishonorable discharge: the oncogenic roles of cleaved E-cadherin fragments. Cancer Res 2012; 72: 2917-23
- Ezzedine K et al. Vitiligo is not a cosmetic disease. JAAD 2015; 73(5):883-5
- Jin T et al. Soluble E-cadherin in systemic lupus erythematosus. J Rheumatol 2013; 40: 1677-82.
- Matsuyoshi N et al. Soluble E-cadherin: a novel cutaneous disease marker. Br J Dermatol 1995; 132: 745-9.
- Natarajan VT et al. IFN-γ signaling maintains skin pigmentation homeostasis through regulation of melanosome maturation. Proc Natl Acad Sci USA. 2014;111:2301-6.
- Symowicz, J. et al. Engagement of collagen-binding integrins promotes matrix metalloproteinase-9-dependent E-cadherin ectodomain shedding in ovarian carcinoma cells. Cancer Res. 67, 2030-2039 (2007).
- Taïeb A, Picardo M. Clinical practice. Vitiligo. N Engl J Med. 2009;360:160-9.
- Wagner RY, et al. Altered E-Cadherin Levels and Distribution in Melanocytes Precede Clinical Manifestations of Vitiligo. J Invest Dermatol. 2015 Jul;135(7):1810-9
- Wang CQF et al. IL-17 and TNF synergistically modulate cytokine expression while suppressing melanogenesis: potential relevance to psoriasis. J Invest Dermatol. 2013;133:2741-52.
- Wang S et al. Interferon-γ induces senescence in normal human melanocytes. PloS One. 2014;9:e93232.
- Webb KC et al. Tumour necrosis factor-α inhibition can stabilize disease in progressive vitiligo. Br J Dermatol. 2015;173:641-50.
- Whitton, M. E. et al. Interventions for vitiligo. Cochrane Database Syst Rev CD003263 (2010).
- Yang L et al. Interferon-gamma Inhibits Melanogenesis and Induces Apoptosis in Melanocytes: A Pivotal Role of CD8+ Cytotoxic T Lymphocytes in Vitiligo. Acta Derm Venereol. 24 juin 2015;95(6):664-70.

## Claims

1. A method of preparing *in vitro* or *ex vivo* a cell culture comprising keratinocytes and melanocytes or a cellular model of depigmenting disorder comprising a step of exposing simultaneously or sequentially a population of cells comprising keratinocytes and melanocytes to IFNγ and TNFα.

2. The method of claim 1, wherein the method comprises a step of exposing cells to a culture medium, wherein the medium comprises IFNγ and TNFα.

3. The method of claim 1 or 2, wherein IFNγ and TNFα are respectively present in a concentration ranging from 1 to 50 ng/ml of medium, preferably are each present at a concentration of 10 ng/ml of medium.

4. The method according to anyone of claims 1 to 3, wherein cells are human cells.

5. The method according to anyone of claims 1 to 4, wherein cells are from a healthy subject or from a patient suffering from a depigmenting disorder or from an inflammatory skin disease where depigmentation is occurring.

6. The method according to claim 5, wherein the depigmenting disorder is associated with an inflammatory skin disease.

7. The method according to claim 6, wherein the inflammatory skin disease is selected from vitiligo, psoriasis, atopic dermatitis, scleroderma, hypomelanose and leukotrichia.

8. The method according to claim 7, wherein the depigmenting disorder is vitiligo.

9. A cell culture obtained by a method according to anyone of claims 1 to 8, wherein the cell culture comprises keratinocytes, melanocytes, IFNγ and TNFα.

10. A cellular model of depigmenting disorder obtained by a method according to anyone of claims 1 to 8, wherein the cellular model comprises keratinocytes, melanocytes and an epidermis, and wherein more than 50% of melanocytes are detached from the basal layer of the epidermis and/or from adjacent keratinocytes.

11. A cell culture or a cellular model of depigmenting disorder obtained by a method according to anyone of claims 5 to 8, wherein cells are from a patient suffering from a depigmenting disorder or from an inflammatory skin disease where depigmentation is occurring.

12. The cellular model of depigmenting disorder according to claim 10 or 11, wherein the model is a skin model, an epidermis model, or a tissue model comprising an epidermis and a dermis and optionally cells of the skin immune system.

13. Use of a cell culture according to claim 9 or 11 or of a cellular model of depigmenting disorder according to any one of claims 10 to 12 for studying *in vitro* or *ex vivo* pigmentation/depigmentation mechanisms and modulators of these mechanisms.

14. Use of a cell culture according to claim 9 or 11, or of a cellular model of depigmenting disorder according to any one of claims 10 to 12 in a method for screening *in vitro* or *ex vivo* pharmaceutically active molecules suitable for preventing, treating or attenuating a depigmenting disorder in a subject in need thereof.

15. A method for screening *in vitro* or *ex vivo* pharmaceutically active molecules suitable for preventing, treating or attenuating a depigmenting disorder in a subject in need thereof, wherein the method comprises a step of exposing a cell culture according to claim 9 or 11 or a cellular model of depigmenting disorder according to any one of claims 10 to 12 to a molecule to be tested and a step of evaluating the ability of the tested molecule to inhibit MMP9 expression or function, to inhibit both MMP9 and Janus kinases (JAK) expression or function, in particular the expression or function of a JAK selected from JAK1, JAK2, JAK3, TYK2 and any combination thereof to induce melanin production and/or melanocyte function, to cleave E-cadherin, to increase soluble E-cadherin expression, to increase cytoplasmic redistribution of E-cadherin, to relocalize membrane bound E-cadherin, and/or to stabilize melanocytes to the basement membrane of the epidermis, said ability(ies) indicating that the tested molecule is a pharmaceutically active molecule suitable for preventing, treating or attenuating a depigmenting disorder in a subject in need thereof.

## Patentansprüche

1. Verfahren zur Herstellung *in vitro* oder *ex vivo* einer Zellkultur, die Keratinozyten und Melanozyten enthält, oder eines zellulären Modells einer Depigmentierungsstörung, umfassend einen Schritt, bei dem eine Zellpopulation, die Keratinozyten und Melanozyten umfasst, gleichzeitig oder nacheinander IFNγ und TNFα ausgesetzt wird.

2. Verfahren nach Anspruch 1, wobei das Verfahren einen Schritt umfasst, bei dem Zellen einem Kulturmedium ausgesetzt werden, wobei das Medium IFNγ und TNFα umfasst.

3. Verfahren nach Anspruch 1 oder 2, wobei IFNγ und TNFα jeweils in einer Konzentration im Bereich von 1 bis 50 ng/ml Medium vorliegen, vorzugsweise jeweils in einer Konzentration von 10 ng/ml Medium.

4. Verfahren nach einem der Ansprüche 1 bis 3, wobei es sich bei den Zellen um menschliche Zellen handelt.

5. Verfahren nach einem der Ansprüche 1 bis 4, wobei die Zellen von einem gesunden Probanden oder von einem Patienten stammen, der an einer Depigmentierungsstörung oder einer entzündlichen Hauterkrankung leidet, bei der eine Depigmentierung auftritt.

6. Verfahren nach Anspruch 5, wobei die Depigmentierungsstörung mit einer entzündlichen Hauterkrankung einhergeht.

7. Verfahren nach Anspruch 6, wobei die entzündliche Hauterkrankung ausgewählt ist aus Vitiligo, Psoriasis, atopischer Dermatitis, Sklerodermie, Hypomelanose und Leukotrichie.

8. Verfahren nach Anspruch 7, wobei die Depigmentierungsstörung Vitiligo ist.

9. Zellkultur, erhalten durch ein Verfahren gemäß einem der Ansprüche 1 bis 8, wobei die Zellkultur Keratinozyten, Melanozyten, IFNγ und TNFα umfasst.

10. Zelluläres Modell einer Depigmentierungsstörung, erhalten durch ein Verfahren nach einem der Ansprüche 1 bis 8, wobei das zelluläre Modell Keratinozyten, Melanozyten und eine Epidermis umfasst und wobei mehr als 50 % der Melanozyten von der Basalschicht der Epidermis abgelöst sind und/oder von benachbarten Keratinozyten.

11. Zellkultur oder zelluläres Modell einer Depigmentierungsstörung, erhalten durch ein Verfahren nach einem der Ansprüche 5 bis 8, wobei die Zellen von einem Patienten stammen, der an einer Depigmentierungsstörung oder einer entzündlichen Hauterkrankung leidet, bei der eine Depigmentierung auftritt.

12. Zelluläres Modell einer Depigmentierungsstörung nach Anspruch 10 oder 11, wobei das Modell ein Hautmodell, ein Epidermismodell oder ein Gewebemodell ist, das eine Epidermis und eine Dermis und optional Zellen des Hautimmunsystems umfasst.

13. Verwendung einer Zellkultur nach Anspruch 9 oder 11 oder eines zellulären Modells einer Depigmentierungsstörung nach einem der Ansprüche 10 bis 12 zur *in vitro* oder *ex vivo* Untersuchung von Pigmentierungs-/Depigmentierungsmechanismen und Modulatoren dieser Mechanismen.

14. Verwendung einer Zellkultur nach Anspruch 9 oder 11 oder eines zellulären Modells einer Depigmentierungsstörung nach einem der Ansprüche 10 bis 12 in einem Verfahren zum *in vitro* oder *ex vivo* Screening pharmazeutisch aktiver Moleküle, die zur Vorbeugung, Behandlung oder Linderung einer Depigmentierungsstörung in einem Patienten, der dies benötigt, geeignet sind.

15. Verfahren zum Screening pharmazeutisch aktiver Moleküle *in vitro* oder *ex vivo,* die zur Vorbeugung, Behandlung oder Linderung einer Depigmentierungsstörung in einem Patienten, der dies benötigt, geeignet sind, wobei das Verfahren einen Schritt das Inkontaktbringen einer Zellkultur nach Anspruch 9 oder 11 oder eines zellulären Modells einer Depigmentierungsstörung nach einem der Ansprüche 10 bis 12 mit einem zu testenden Molekül umfasst und einen Schritt zur Bewertung der Fähigkeit des getesteten Moleküls, die MMP9-Expression oder -Funktion zu hemmen, sowohl die MMP9- als auch die Janus-Kinasen (JAK)-Expression oder -Funktion zu hemmen, insbesondere die Expression oder Funktion eines JAK, ausgewählt aus JAK1, JAK2, JAK3, TYK2 und einer beliebigen Kombination davon, um die Melaninproduktion und/oder Melanozytenfunktion zu induzieren, E-Cadherin zu spalten, die Expression von löslichem E-Cadherin zu erhöhen und die zytoplasmatische Umverteilung von E-Cadherin zu erhöhen, um membrangebundenes E-Cadherin zu relokalisieren und/oder um Melanozyten an der Basalmembran der Epidermis zu stabilisieren, wobei diese Fähigkeit(en) anzeigt(en), dass das getestete Molekül ein pharmazeutisch aktives Molekül ist, das zur Vorbeugung, Behandlung oder Linderung einer Depigmentierungsstörung in einem Patienten, der dies benöigt, geeignet ist.

## Revendications

1. Méthode de préparation *in vitro* ou *ex vivo* d'une culture cellulaire comprenant des kératinocytes et des mélanocytes ou d'un modèle cellulaire de trouble de dépigmentation, comprenant une étape d'exposition simultanée ou séquentielle d'une population de cellules comprenant des kératinocytes et des mélanocytes à de l'IFNγ et du TNFα.

2. Méthode selon la revendication 1, laquelle la méthode comprend une étape d'exposition de cellules à un milieu de culture, dans laquelle le milieu comprend de l'IFNγ et du TNFα.

3. Méthode selon la revendication 1 ou 2, dans laquelle les IFNγ et TNFα sont respectivement présents à une concentration située dans la plage allant de 1 à 50 ng/ml de milieu, de préférence sont présents chacun à une concentration de 10 ng/ml de milieu.

4. Méthode selon l'une quelconque des revendications 1 à 3, dans laquelle les cellules sont des cellules humaines.

5. Méthode selon l'une quelconque des revendications 1 à 4, dans laquelle les cellules proviennent d'un sujet en bonne santé ou d'un patient souffrant d'un trouble de dépigmentation ou d'une maladie cutanée inflammatoire où une dépigmentation survient.

6. Méthode selon la revendication 5, dans laquelle le trouble de dépigmentation est associé à une maladie cutanée inflammatoire.

7. Méthode selon la revendication 6, dans laquelle la maladie cutanée inflammatoire est sélectionnée parmi un vitiligo, un psoriasis, une dermatite atopique, une sclérodermie, une hypomélanose et une leucotrichie.

8. Méthode selon la revendication 7, dans laquelle le trouble de dépigmentation est un vitiligo.

9. Culture cellulaire obtenue par une méthode selon l'une quelconque des revendications 1 à 8, laquelle culture cellulaire comprend des kératinocytes, des mélanocytes, de l'IFNγ et du TNFα.

10. Modèle cellulaire de trouble de dépigmentation obtenu par une méthode selon l'une quelconque des revendications 1 à 8, lequel modèle cellulaire comprend des kératinocytes, des mélanocytes et un épiderme, et dans lequel plus de 50 % des mélanocytes sont détachés de la couche basale de l'épiderme et/ou de kératinocytes adjacents.

11. Culture cellulaire ou modèle cellulaire de trouble de dépigmentation obtenu par une méthode selon l'une quelconque des revendications 5 à 8, dans lequel les cellules proviennent d'un patient souffrant d'un trouble de dépigmentation ou d'une maladie cutanée inflammatoire où une dépigmentation survient.

12. Modèle cellulaire de trouble de dépigmentation selon la revendication 10 ou 11, lequel modèle est un modèle de peau, un modèle d'épiderme, ou un modèle de tissu comprenant un épiderme et un derme et éventuellement des cellules du système immunitaire cutané.

13. Utilisation d'une culture cellulaire selon la revendication 9 ou 11 ou d'un modèle cellulaire de trouble de dépigmentation selon l'une quelconque des revendications 10 à 12 pour étudier *in vitro* ou *ex vivo* des mécanismes de pigmentation/dépigmentation et des modulateurs de ces mécanismes.

14. Utilisation d'une culture cellulaire selon la revendication 9 ou 11 ou d'un modèle cellulaire de trouble de dépigmentation selon l'une quelconque des revendications 10 à 12 dans une méthode de criblage *in vitro* ou *ex vivo* de molécules actives sur le plan pharmaceutique convenant pour prévenir, traiter ou atténuer un trouble de dépigmentation chez un sujet en ayant besoin.

15. Méthode de criblage *in vitro* ou *ex vivo* des molécules actives sur le plan pharmaceutique convenant pour prévenir, traiter ou atténuer un trouble de dépigmentation chez un sujet en ayant besoin, laquelle méthode comprend une étape d'exposition d'une culture cellulaire selon la revendication 9 ou 11 ou d'un modèle cellulaire de trouble de dépigmentation selon l'une quelconque des revendications 10 à 12 à une molécule devant être testée, et une étape d'évaluation de l'aptitude de la molécule testée à inhiber l'expression ou la fonction de MMP9, à inhiber l'expression ou la fonction à la fois de MMP9 et des Janus kinases (JAK), en particulier l'expression ou la fonction d'une JAK sélectionnée parmi JAK1, JAK2, JAK3, TYK2 et l'une quelconque de leurs combinaisons pour induire la production de mélanine et/ou la fonction des mélanocytes, pour couper la E-cadhérine, pour augmenter l'expression d'E-cadhérine soluble, pour augmenter la redistribution cytoplasmique d'E-cadhérine, pour relocaliser l'E-cadhérine liée à la membrane, et/ou pour stabiliser les mélanocytes à la membrane de base de l'épiderme, ladite ou lesdites aptitudes indiquant que la molécule testée est une molécule active sur le plan pharmaceutiquepour prévenir, traiter ou atténuer un trouble de dépigmentation chez un sujet en ayant besoin.
